Europäisches Patentamt

**European Patent Office**

Office européen des brevets

(19)

(11) Publication number: **0 211 976**
**B1**

## EUROPEAN PATENT SPECIFICATION

(12)

(45) Date of publication of the patent specification:
**15.11.89**

(51) Int. Cl.⁴: **B29C 47/02, A61B 1/00**

(21) Application number: **85110149.3**

(22) Date of filing: **13.08.85**

(54) Method for producing an optical sensor.

(43) Date of publication of application:
**04.03.87 Bulletin 87/10**

(45) Publication of the grant of the patent:
**15.11.89 Bulletin 89/46**

(84) Designated Contracting States:
**DE FR GB**

(56) References cited:
**EP-A- 0 097 934**
**EP-A- 0 100 517**
**FR-A- 2 365 999**
**GB-A- 1 526 777**

**PATENT ABSTRACTS OF JAPAN, vol. 9,
no. 187 (M-401), 3rd August 1985, page 145 M 401; & JP-A- 60 54830 (SUMITOMO DENKI KOGYO K.K.) 29-03-1985**
**PATENT ABSTRACTS OF JAPAN, vol. 9,
no. 187 (M-401), 3rd August 1985, page 146 M 401; & JP-A- 60 54833 (SUMITOMO DENKI KOGYO K.K.) 29-03-1985**

(73) Proprietor: **SUMITOMO ELECTRIC INDUSTRIES LIMITED, No. 15, Kitahama 5-chome Higashi-ku, Osaka-shi Osaka 541(JP)**

(72) Inventor: **Ueba, Yoshinobu c/o Osaka Works of, Sumitomo Electric Ind. Ltd. 1-3, Shimaya 1-chome, Konohana-ku Osaka-shi Osaka-fu(JP)**
Inventor: **Matsumiya, Norifumi c/o Osaka Works of, Sumitomo Electric Ind. Ltd. 1-3, Shimaya 1-chome, Konohana-ku Osaka-shi Osaka-fu(JP)**
Inventor: **Matsubara, Hironaga c/o Osaka Works of, Sumitomo Electric Ind. Ltd. 1-3, Shimaya 1-chome, Konohana-ku Osaka-shi Osaka-fu(JP)**

(74) Representative: **Eitle, Werner, Dipl.-Ing. et al, Hoffmann, Eitle & Partner Patentanwälte Arabellastrasse 4, D-8000 München 81(DE)**

## Description

### FIELD OF THE INVENTION

The present invention relates to a method for producing an optical sensor. More particularly, it relates to a method for producing an optical sensor comprising a core acting as an optical waveguide part for illumination, a cladding surrounding the core and having a refractive index lower than that of the core and an image fiber which is inserted in an elongate bore formed in the core along the entire length of the sensor, or embedded in the core.

### BACKGROUND OF THE INVENTION

An optical sensor is a light- or image-transmitting elongate element comprising an optical waveguide, an image fiber and optionally an elongate hollow tube member for transporting a fluid.

Such the optical sensor is often used as a light transmitting element of an endoscope for inspecting an interior of an organ such as an abdomen, a heart or a blood vessel. The optical sensor is introduced in a human or mammal body to be inspected together with an image pick up adapter which comprises a lens and forms an image of the organ interior on a front end of the image fiber. To the other end near an operator, other elements of the endoscope are attached. The endoscope comprises an image-receiving apparatus for monitoring the image propagated through the image fiber. The image is directly observed by means of an orthoptic adaptor or projected on a monitoring television. Further, a light source attached at the near end of the optical waveguide emits light, which is propagated through the optical waveguide and illuminates the organ interior.

The conventional optical waveguide comprises bundled transparent plastic optical fibers each having a round cross section. However, gaps are formed between the adjacent optical fibers, and light is not propagated through the gaps. This reduces an effective volume of the optical waveguide.

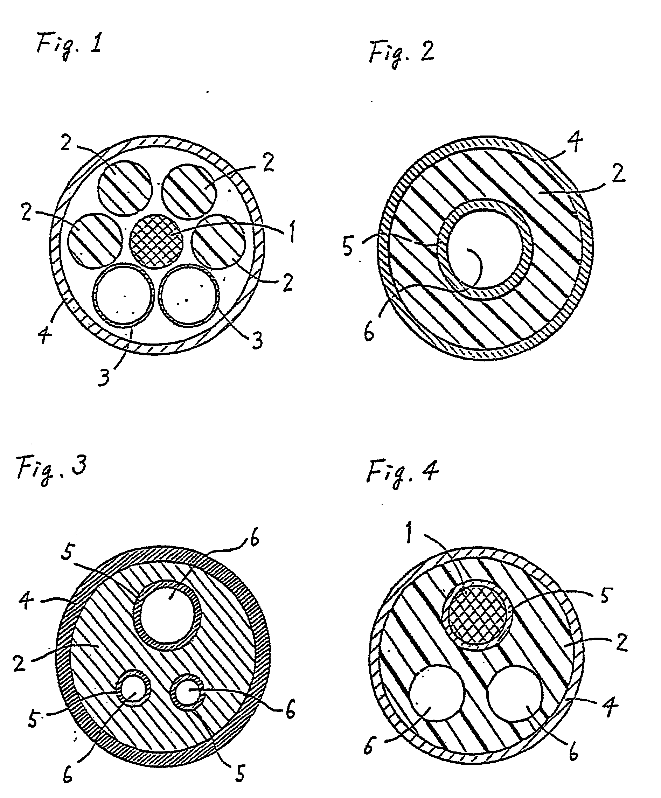

A hollow tube is additionally assembled in the optical sensor to remove a fluids such as blood from the organ interior or to inject physiologic saline in a small balloon attached to the front end of the sensor so as to inflate the balloon and exclude the blood from a part to be observed. Air in the balloon is exhausted through an additional tube provided in the sensor. Fig. 1 shows a conventional sensor having an image fiber 1, plural optical fibers 2 as optical waveguides and two tubes 3 all of which are enclosed in a cladding 4. Such the sensor also has gaps between the waveguides and/or the tubes.

It has been proposed to increase the effective volume of the optical waveguide by utilizing all the cross sectional area of the sensor except the cross sections of the image fiber and/or the tubes as the optical waveguide (cf. Japanese Patent Kokai Publication (unexamined) Nos. 54830/1985 and 54833/1985 both published on 29. 03. 85). For example, the sensor containing the optical waveguide and the image fiber has a cross section as shown in Fig. 2, 3 or 4. The sensor of Fig. 2 has one bore 6 having a cladding layer 5 through which an image fiber is inserted, that of Fig. 2 has three bores 6 each having a cladding layer 5 through one of which an image fiber is inserted, and that of Fig. 4 has one image fiber 1 and two vacant bores 6.

In the methods of the above Publications, the bore is formed in the waveguide by extruding a core material from a coextrusion die with supplying at least one metal wire, simultaneously extruding a cladding material around the core material to form a cylindrical waveguide with the metal wire embedded and removing the metal wire after stretching it to form a bore in the core.

These methods, however, have some drawbacks. For example, the inner diameter of the die and the outer diameter of the metal wire should be extremely precise since the former determines the outer diameter of the waveguide and the latter determines the inner diameter of the bore. The methods include many steps including the removal of the metal wire. Further, marks are generated on the inner surface of the bore during the removal of the metal wire. Since the inner surface of the bore should be coated with a material having a smaller refractive index than that of the waveguide (core) material so as to reflect light to be transmitted, the metal wire should be coated by such the material before extrusion of the core material. This makes the structure of the die more complicate.

EP-A-0 097 934 describes a method of producing an optical fiber with an image fiber and cladding as well as a bore, which is formed by dual in line extrusion using two independent dies. By such a method it is difficult to form a bore having a uniform diameter along its length. In particular, the method imposes severe limitations on the viscosity of material surrounding the bore.

### SUMMARY OF THE INVENTION

One object of the present invention is to provide an improved method for producing an optical sensor comprising a core acting as an optical waveguide part, a cladding surrounding the core and an image fiber inserted in a bore formed in the core.

Another object of the present invention is to provide an improved method for producing an optical sensor having an enlarged effective cross sectional area of the optical waveguide.

Further object of the present invention is to provide a novel optical sensor comprising a core acting as an optical waveguide part and having a bore extending along the entire length of the sensor, a cladding surrounding the core, and an image fiber embedded in the core.

The objects of the present invention are solved by a method for producing an optical sensor comprising a core acting as an optical waveguide part, a cladding surrounding the core and at least one image fiber inserted in at least one bore formed in the core, which method comprising forming an optical waveguide comprising a core in which at least one bore is extended along its entire length and a cladding surrounding the core and inserting the image fiber in the bore, characterized in that the optical waveguide is produced by a method comprising coextruding core and cladding materials with blowing air in the core material to form the bore in the core, or a method comprising coextruding core and cladding materials with supplying at least one metal wire in the core material, removing the metal wire from the core after stretching the wire to form the bore in the core and drawing the waveguide at a temperature higher than the glass transition temperature of the core material, or a method comprising coextruding core and cladding materials with supplying at least one hollow fiber in the core to form the bore in the core.

The objects of the present invention are further solved by an optical sensor comprising a core acting as an optical waveguide part and having at least one bore extending along the entire length of the sensor, a cladding surrounding said core, at least one image fiber embedded in said core or inserted in said at least one bore, core and cladding materials being coextruded while supplying said at least one image fiber and at least one hollow fiber in said core to form said bore in said core.

## BRIEF DESCRIPTION OF THE DRAWINGS

Fig. 1 shows a cross section of the conventional optical sensor comprising plural optical fibers as optical waveguides, an image fiber and two hollow tubes and a cladding surrounding all these elongate elements,

Figs. 2, 3 and 4 show cross sections of embodiments of an optical sensor to be produced according to the present invention,

Fig. 5 shows a cross section of a die to be used in the first embodiment of the method according to the present invention,

Fig. 6 schematically shows an equipment used in the first embodiment of the method according to the present invention,

Figs. 7 and 8 schematically show equipments used in the second embodiment of the method according to the present invention,

Fig. 9 schematically shows an equipment used in the third embodiment of the method according to the present invention,

Fig. 10 shows a cross section of a die to be used in the third embodiment of the method according to the present invention, and

Fig. 11 shows a cross section of an optical sensor produced by the third embodiment of the method according to the present invention.

## DETAILED DESCRIPTION OF THE INVENTION

The core of the sensor to be produced by the method of the invention is usually made of a transparent amorphous polymeric material optionally added with a plasticizer. Specific examples of the core material are homo- or co-polymer of styrene or methacrylate (e.g., polymethyl methacrylate, n-butyl methacrylate/isobutyl methacrylate copolymer and the like), polymethyl methacrylate plasticized with adipate, and polycarbonate.

The core is surrounded by a cladding to confine the propagated light in the core. The cladding is made of a material having a smaller refractive index than that of the core material such as fluororesin (e.g., homo- or co-polymer of vinylidene fluoride or tetrafluoroethylene), silicone resin and ethylene/vinyl acetate copolymer.

Preferably, the optical sensor has an outer diameter of 0.25 to 2.2 mm, the cladding has a thickness of 0.01 to 0.04, and the bore has a diameter up to 1.5 mm.

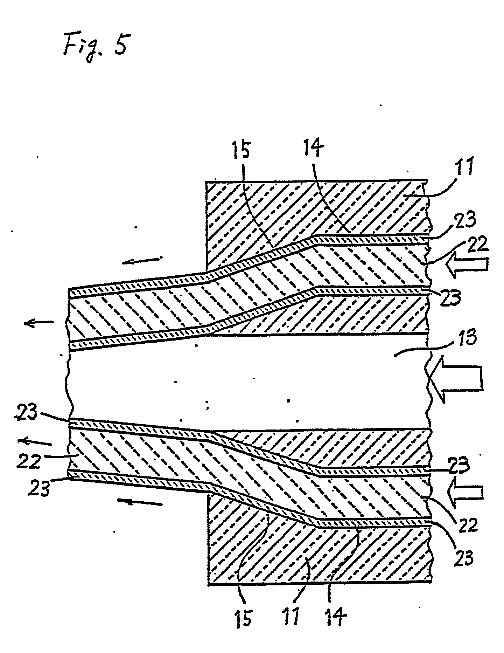

In the first embodiment of the method according to the present invention, the optical waveguide is produced by means of a coextrusion die shown in Fig. 5.

The die 11 has an aperture 13 and an inlet 14 for the core and cladding materials coaxially surrounding the aperture 13.

At the front end of the inlet 14, a frustoconical slant channel 15 is provided with. The tip of the channel 15 opens near the front end of the aperture 13.

The core material 22 and the cladding material 23 are fed into the inlet 14. Simultaneously, a suitable volume of air is injected through the aperture 13 at suitable pressure.

The core and cladding materials are coextruded in the form of a tube a central hollow portion of which is formed by injected air.

The thus produced optical waveguide has a cross section as shown in Fig. 2, wherein the inner and outer surfaces of the core 2 are coated by the cladding material 4,5. Through the bore 6, an image fiber is inserted to form an optical sensor.

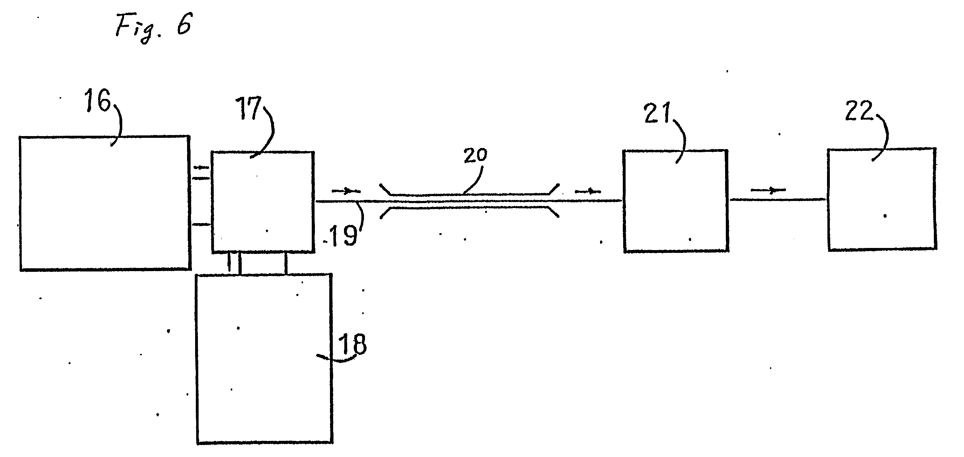

Fig. 6 schematically shows an equipment for producing the optical waveguide of Fig. 2.

The core and cladding materials are fed in a coextrusion die part 17 having the die 11 of Fig. 5 from extruders 16 and 18, respectively. A fiber 19 extruded from the die is cooled in a cooling zone 20, and drawn by a capstan puller 21 and finally wound by a winder 22.

The outer diameter of the fiber 19 is controlled by adjusting the puling rate of the capstan 21 and the fed amounts of the core and cladding materials.

A waveguide having two or more bores in the core can be produced by means of a suitable die having two or more apertures for injecting air.

In the second embodiment of the method of the present invention, the optical waveguide is produced by coextruding the core and cladding materials with supplying at least one metal wire in the core material to form an optical waveguide having the metal wire embedded, removing the wire from the fiber after stretching it and drawing the waveguide at a temperature higher than a glass transition temperature of the core material.

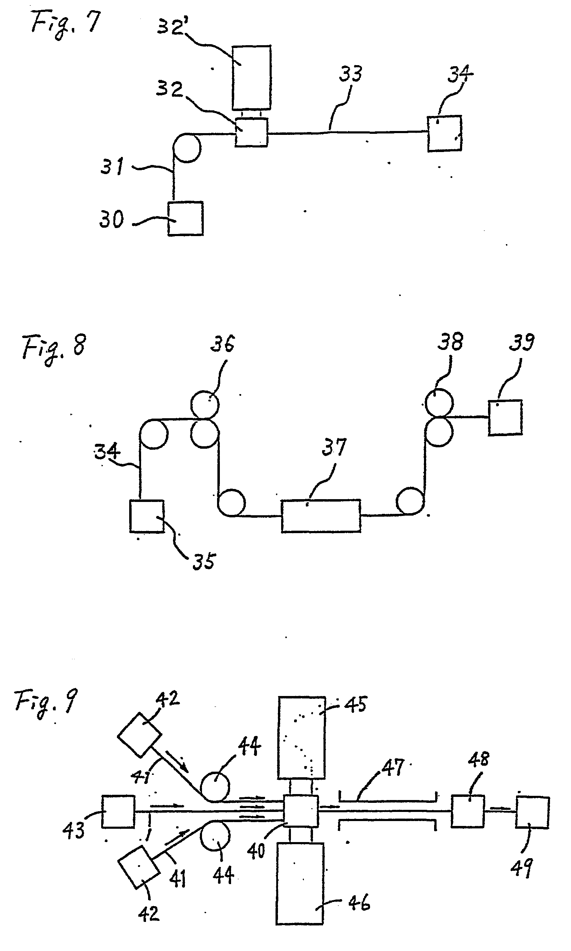

Such the method will be described with reference to Figs. 7 and 8.

In Fig. 7, a metal wire 31 is supplied from a supplier 30 to a die 32, and the core and cladding materials are supplied from an extruder 32' and coated around the metal wire 31. Two or more metal wires may be supplied to finally produce an optical waveguide having two or more bores. An optical waveguide 33 with the metal wire is wound by a winder 34. In a subsequent step, the metal wire is removed from the optical waveguide.

In Fig. 8, the optical waveguide 34 without the wire is unwound from a supplier 35 and wound by a winder 39 through a roll 35 for controlling a supply rate, a heating means 37 and a roll 38 for controlling a winding rate. The heating means 37 may be a furnace, a water or oil bath or a heating roll. The optical waveguide is heated at a temperature higher than the glass transition temperature of the core material by 10 to 50°C. The drawn rate of the optical waveguide is controlled by adjusting the supply rate and the winding rate. Usually, the waveguide is drawn by a factor of 2 to 3.

The metal wire is made of a rigid flexible metal such as annealed copper wire.

In the third embodiment of the method of the present invention, the waveguide is produced by substantially the same manner as in the second embodiment but using a hollow fiber in place of the metal wire and not drawing the waveguide. In this embodiment, the hollow fiber forms the bore of the optical waveguide.

The hollow fiber is made of a thermoplastic resin (e.g, polyamide, polysulfone, polyethersulfone, polyetherimide, polyvinylidene fluoride, polytetrafluoroethylene, tetrafluoroethylene/hexafluoropropylene copolymer and the like), a thermoset resin (e.g., polyesterimide, polyamideimide, polyimide and the like), quartz glass, multicomponent glass, metal (e.g., gold, silver, copper, aluminum, stainless steel and the like). Because of flexibility, the resins are preferable.

In this embodiment, an image fiber may be supplied together with the hollow fiber.

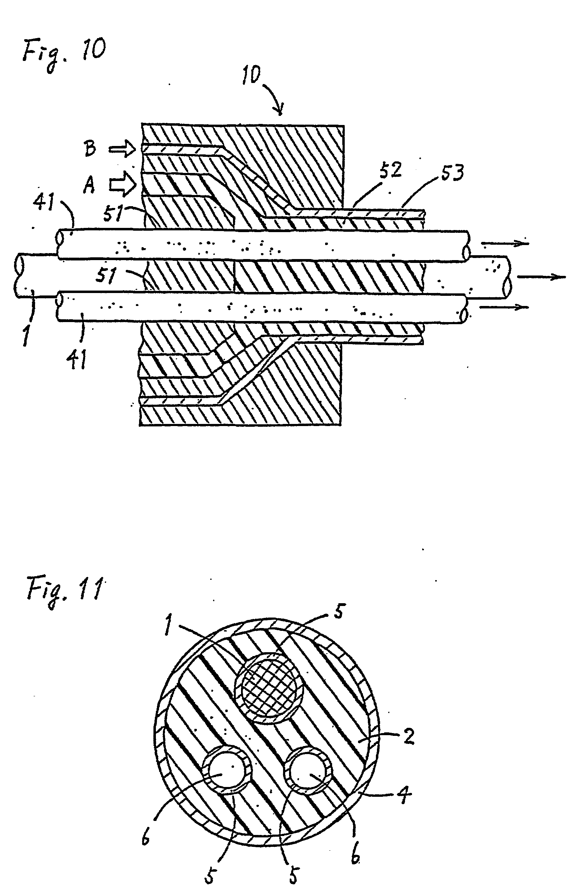

This method is carried out by an equipment schematically shown in Fig. 9.

Two hollow fibers 41 are continuously supplied from corresponding hollow fiber suppliers 42 to a coextrusion die 40. An image fiber 1 is continuously supplied from an image fiber supplier 43 to the coextrusion die 1. Distance between the two hollow fibers 7 is controlled by means of a pair of guide rolls 44.

The coextrusion die is attached to a head of an extruder consisting of extruders 45,46 for the core material and the cladding material, respectively.

Fig. 10 shows an enlarged cross section of a die 10, which has apertures 51 for forwarding the respective hollow fiber and an aperture (not shown) for forwarding the image fiber 1. In the die 10, the core material A is cylindrically extruded around the hollow fibers 41 and the image fiber 1 and then a cladding material B is applied around the core material to form an optical waveguide comprising a core 52 and a cladding 53. Then, the cladding is sized to form an optical sensor of Fig. 11.

The optical sensor extruded from the die 10 is cooled in a cooling zone 47 to solidify the cladding material. In the downstream of the cooling zone, a capstan puller 48 and a winder 49 are provided with.

Extrusion conditions are so selected that the thermal deformation of the hollow fiber is prevented during extrusion of the core and cladding materials. For example, the hollow fiber is made of a material having a deformation temperature higher than the extrusion temperature of the core material preferably by at least 10°C.

This method can be modified by replacing the image fiber with a third hollow fiber. In this case, the image fiber is inserted in one of three hollow fibers. This modification is preferred since the image fiber is not subjected to a high temperature so that it is not thermally damaged.

The present invention will be explained by following Examples.

EXAMPLE 1

By means of the die of Fig. 5, an optical waveguide shown in Fig. 2 having one bore was produced.

As the core and cladding materials, polymethyl methacrylate and vinylidene fluoride/tetrafluoroethylene copolymer were used, respectively.

The optical waveguide was produced under following conditions:

4

| Extrusion rate of the resin: | 20–40 cm$^3$/min. |
| Pulling rate: | 5–10 m/min. |
| Extrusion temperature: | 240°C |

The produced waveguide had an outer diameter of 2.2 mm, a bore diameter of 1.5 mm and cladding thickness of 20μm.

EXAMPLE 2

In the same manner as in EXAMPLE 1 but using, as a core material, n-butyl methacrylate/isobutyl methacrylate copolymer or polymethyl methacrylate plasticized with adipate and, as a cladding material, polymer of fluorine-containing alkyl methacrylate, an optical waveguide was produced under substantially the same conditions but the extrusion temperature being 150-200°C.

EXAMPLE 3

A core material of polymethyl methacrylate (glass transition temperature, 105°C) and a cladding material of vinylidene fluoride/tetrafluoroethylene copolymer were extruded and applied around annealed copper wire having an outer diameter of 1.06 mm. Then, the copper wire was removed to form an optical waveguide having an outer diameter of 1.70 mm and a bore diameter of 1.06 mm.

The produced optical waveguide was drawn with a draw ratio of two in a furnace kept at 140°C to produce the waveguide having an outer diameter of 1.2 mm and a bore diameter of 0.75 mm. Its physical properties are shown in Table.

EXAMPLE 4

In the same manner as in EXAMPLE 3 but drawing a waveguide having an outer diameter of 2.08 mm and a bore diameter of 1.30 mm with a draw ratio of three at 150°C, an optical waveguide having an outer diameter of 1.2 mm and a bore diameter of 0.75 mm was produced. Its physical properties are shown in Table.

COMPARATIVE EXAMPLE

In the same manner as in EXAMPLE 3 but not drawing the waveguide after removing the wire, an optical waveguide having an outer diameter of 1.2 mm and a bore diameter of 0.75 mm was produced. Its physical properties are shown in Table.

Table

| | Example 3 | Example 4 | Comparative Example |
|---|---|---|---|
| Strength[1] (Kg/mm$^2$) | 12.9 | 11.6 | 8.0 |
| Elongation[2] (%) | 110 | 112 | 4.2 |

Note
1) Tensile strength when elongation starts measured by Instron tensile tester.
2) Elongation at break measured by Instron tensile tester.

In these examples, an image sensor could be produced by inserting an image fiber in the bore of the waveguide.

EXAMPLE 5

An optical sensor was produced by means of the die of Fig. 10.

From the die, supplied were two hollow fibers made of polysulfone (manufactured by UCC) each having an outer diameter of 0.6 mm and an inner diameter of 0.5 mm and an image fiber having 3,000 picture elements, a diameter of the picture element bundle of 0.5 mm and an outer diameter of 0.95 mm. Simultaneously, n-butyl methacrylate/isobutyl methacrylate copolymer as a core material and a blend of vinylidene fluoride/tetrafluoroethylene copolymer and polynbutyl methacrylate were extruded to form an optical sensor having an outer diameter of 2.5 mm.

## EXAMPLE 6

In the same manner as in EXAMPLE 5 but using two hollow fiber made of polyamideimide having an outer diameter of 0.6 mm and an inner diameter of 0.5 mm and a hollow fiber made of polyamideimide having an outer diameter of 1.1 mm and an inner diameter of 1.0 mm, an optical waveguide having three bores was produced. Then, an image fiber was inserted in the hollow fiber having the inner diameter of 1.0 mm to produce an optical sensor.

## EXAMPLE 7

In the same manner as in EXAMPLE 5 but using two hollow fiber made of tetrafluoroethylene/hexafluoroethylene copolymer having an outer diameter of 0.6 mm and an inner diameter of 0.5 mm and a hollow fiber made of the same copolymer having an outer diameter of 1.1 mm and an inner diameter of 1.0 mm, an optical waveguide was produced. Then, an image fiber was inserted in the hollow fiber having the inner diameter of 1.0 mm to produce an optical sensor.

**Claims**

1. A method for producing an optical sensor comprising a core (2) acting as an optical waveguide part, a cladding (4, 5) surrounding the core and at least one image fiber (1) inserted in at least one bore (6) formed in the core (2), which method comprising forming an optical waveguide comprising a core (2) in which at least one bore (6) is extended along its entire length and a cladding (4, 5) surrounding the core (2) and inserting the image fiber (1) in the bore (6), characterized in that the optical waveguide is produced by:

a method comprising coextruding core and cladding materials with blowing air in the core material to form the bore (6) in the core (2), or

a method comprising coextruding core and cladding materials with supplying at least one metal wire (31) in the core material, removing the metal wire (31) from the core (2) after stretching the wire (31) to form the bore (6) in the core (2) and drawing the waveguide at a temperature higher than the glass transition temperature of the core material, or

a method comprising coextruding core and cladding materials with supplying at least one hollow fiber in the core (2) to form the bore (6) in the core (2).

2. A method according to claim 1, wherein the optical waveguide is produced by a method comprising coextruding core and cladding materials with blowing air in the core material to form the bore (6) in the core (2).

3. A method according to claim 1, wherein the optical waveguide is produced by a method comprising coextruding core and cladding materials with supplying the metal wire (31) in the core material, removing the metal wire (31) from the core (2) after stretching the wire (31) to form the bore (6) in the core (2) and drawing the waveguide at a temperature higher than the glass transition temperature of the core material.

4. A method according to claim 3, wherein the drawing temperature is higher than the glass transition temperature of the core material by 10 to 50°C.

5. A method according to claim 3, wherein the draw ratio of the waveguide is 2 to 3.

6. A method according to claim 1, wherein the waveguide is produced by a method comprising coextruding core an cladding materials with supplying the hollow fiber in the core (2) to form the bore (6) in the core (2).

7. An optical sensor comprising a core (2) acting as an optical waveguide part and having at least one bore (6) extending along the entire length of the sensor, a cladding (4, 5) surrounding said core (2), at least one image fiber (1) embedded in said core (2) or inserted in said at least one bore (6), core and cladding materials being coextruded while supplying said at least one image fiber (1) and at least one hollow fiber in said core (2) to form said bore (6) in said core (2).

**Patentansprüche**

1. Verfahren zur Herstellung eines optischen Sensors mit einem als ein optisches Wellenleiterteil dienenden Kern (2), einer den Kern umgebenden Umhüllung (4, 5) und mindestens einer in wenigstens eine in dem Kern (2) gebildete Bohrung (6) eingefügten Bildfaser (1), welches Verfahren folgende Schritte enthält:

Bilden eines optischen Wellenleiters mit einem Kern (2), bei welchem sich wenigstens eine Bohrung (6) und eine den Kern (2) umgebende Umhüllung (4, 5) entlang der gesamten Länge erstreckt, und Einfügen der Bildfaser (1) in die Bohrung (6), dadurch gekennzeichnet, daß der optische Wellenleiter hergestellt wird durch:

ein Verfahren bestehend aus Koextrudieren von Kern- und Umhüllungsmaterialien mit Einblasen von Luft in das Kernmaterial, um die Bohrung (6) in dem Kern (2) zu bilden, oder ein Verfahren bestehend aus Koextrudieren von Kern- und Umhüllungsmaterialien mit Zuführen von wenigstens einem Metalldraht (31) in das Kernmaterial, Entfernen des Metalldrahtes (31) aus dem Kern (2) nach Dehnen des

Drahts, um die Bohrung (6) in dem Kern (2) zu bilden, und Ziehen des Wellenleiters bei einer Temperatur höher als die Glasübergangstemperatur des Kernmaterials, oder

ein Verfahren bestehend aus Koextrudieren von Kern- und Umhüllungsmaterialien mit Zuführen mindestens einer hohlen Faser in den Kern (2), um die Bohrung (6) in dem Kern (2) zu bilden.

2. Verfahren nach Anspruch 1, dadurch gekennzeichnet, daß der optische Wellenleiter durch ein Verfahren hergestellt wird, welches Koextrudieren von Kern- und Umhüllungsmaterialien mit Einblasen von Luft in das Kernmaterial enthält, um die Bohrung (6) in dem Kern (2) zu bilden.

3. Verfahren nach Anspruch 1, dadurch gekennzeichnet, daß der optische Wellenleiter durch ein Verfahren hergestellt wird, welches Koextrudieren von Kern- und Umhüllungsmaterialien mit Zuführen des Metalldrahts (31) in das Kernmaterial, Entfernen des Metalldrahts (31) von dem Kern (2) nach Dehnen des Drahts (31), um die Bohrung (6) in dem Kern (2) zu bilden und Ziehen des Wellenleiters bei einer Temperatur höher als die Glasübergangstemperatur des Kernmaterials enthält.

4. Verfahren nach Anspruch 3, dadurch gekennzeichnet, daß die Ziehtemperatur um 10 bis 50°C höher als die Glasübergangstemperatur des Kernmaterials ist.

5. Verfahren nach Anspruch 3, dadurch gekennzeichnet, daß das Ziehverhältnis des Wellenleiters 2 bis 3 ist,

6. Verfahren nach Anspruch 1, dadurch gekennzeichnet, daß der Wellenleiter durch ein Verfahren hergestellt wird, welches Koextrudieren von Kern- und Umhüllungsmaterialien mit Zuführen der hohlen Faser in den Kern (2) enthält, um die Bohrung (6) in dem Kern (2) zu bilden.

7. Optischer Sensor mit einem als ein optisches Wellenleiterteil dienenden Kern (2) und mit wenigstens einer Bohrung (6), die sich entlang der gesamten Länge des Sensors erstreckt, einer den Kern (2) umgebenden Umhüllung (4, 5), wenigstens einer in den Kern (2) eingebetteten oder in die wenigstens eine Bohrung (6) eingefügten Bildfaser (1), wobei Kern- und Umhüllungsmaterialien koextrudiert werden, während die wenigstens eine Bildfaser (1) und wenigstens eine hohle Faser in dem Kern (2) zugeführt werden, um die Bohrung (6) in dem Kern (2) zu bilden.

**Revendications**

1. Procédé permettant de produire un capteur optique qui comprend une âme (2) faisant fonction de partie guide d'onde optique, un revêtement (4, 5) entourant l'âme et au moins une fibre (1) de transmission d'image qui est insérée dans au moins un trou (6) formé dans l'âme (2), lequel procédé consiste à former un guide d'onde optique comprenant une âme (2) dans laquelle au moins un trou (6) s'étend sur toute sa longueur et un revêtement (4, 5) entourant le trou (2) et à insérer la fibre de transmission d'image (1) dans le trou (6), caractérisé en ce que le guide d'onde optique est produit par:

un procédé consistant à effectuer l'extrusion, en couches multiples, de matériaux d'âme et de revêtement en insufflant de l'air dans le matériau de l'âme pour former le trou (6) dans l'âme (2), ou bien

un procédé consistant à effectuer l'extrusion, en couches multiples, de matériaux d'âme et de revêtement en fournissant au moins un fil métallique (31) dans le matériau de l'âme, à retirer de l'âme (2) le fil métallique (31) après avoir tendu le fil métallique (31) afin de former le trou (6) dans l'âme (2) et à étirer le guide d'onde à une température supérieure à la température de transition vitreuse du matériau de l'âme, ou bien

un procécé consistant à effectuer l'extrusion, en couches multiples, de matériaux d'âme et de revêtement en fournissant au moins une fibre creuse dans l'âme (2) afin de former le trou (6) dans l'âme.

2. Procédé selon la revendication 1, où on produit le guide d'onde optique par un procédé consistant à effectuer l'extrusion, en couches multiples, de matériaux d'âme et de revêtement en insufflant de l'air dans le matériau de l'âme pour former le trou (6) dans l'âme (2).

3. Procédé selon la revendication 1, où on produit le guide d'onde optique par un procédé consistant à effectuer l'extrusion, en couches multiples, de matériaux d'âme et de revêtement en fournissant le fil métallique (31) dans le matériau de l'âme, à retirer de l'âme (2) le fil métallique (31) après avoir tendu le fil (31) pour former le trou (6) dans l'âme (2) et à étirer le guide d'onde à une température supérieure à la température de transition vitreuse du matériau de l'âme.

4. Procédé selon la revendication 3, où la température d'étirage est supérieure de 10 à 50°C à la température de transition vitreuse du matériau de l'âme.

5. Procédé selon la revendication 3, où le taux d'étirage du guide d'onde est de 2 à 3.

6. Procédé selon la revendication 1, où on produit le guide d'onde par un procédé à effectuer l'extrusion, en couches multiples, de matériaux d'âme et de revêtement en fournissant la fibre creuse dans l'âme (2) afin de former le trou (6) dans l'âme (2) afin de former le trou (6) dans l'âme (2).

7. Capteur optique comprenant une âme (2) faisant fonction de partie guide d'onde optique et possédant au moins un trou (6) qui s'étend sur toute la longueur du capteur, un revêtement (4, 5) entourant ladite âme (2), au moins une fibre de transmission d'image (1) encastrée dans ladite âme (2) ou insérée dans ledit ou lesdits trous (6), les matériaux de l'âme et du revêtement étant extrudés en couches multiples en même temps qu'on fournit ladite ou lesdites fibres de transmission d'image (1) et au moins une fibre creuse dans ladite âme (2) pour former ledit trou (6) dans ladite âme (2).

Fig. 1

Fig. 2

Fig. 3

Fig. 4

Fig. 5

Fig. 6

EP 0 211 976 B1

Fig. 7

Fig. 8

Fig. 9

Fig. 10

Fig. 11